## Europäisches Patentamt
## European Patent Office
## Office européen des brevets

(11) Publication number: **0 112 094**
**B1**

(12) # EUROPEAN PATENT SPECIFICATION

(45) Date of publication of patent specification: **03.06.87**

(51) Int. Cl.⁴: **A 61 M 1/34**

(21) Application number: **83307302.6**

(22) Date of filing: **30.11.83**

(54) Apparatus for blood treatment.

(30) Priority: **30.11.82 JP 210048/82**
**30.12.82 JP 231247/82**

(43) Date of publication of application:
**27.06.84 Bulletin 84/26**

(45) Publication of the grant of the patent:
**03.06.87 Bulletin 87/23**

(84) Designated Contracting States:
**DE FR GB IT**

(56) References cited:
EP-A-0 046 126
EP-A-0 052 004
FR-A-2 242 112
US-A-3 570 672
US-A-3 640 393
US-A-4 013 564
US-A-4 071 444

(73) Proprietor: **Takeda Chemical Industries, Ltd.**
**27, Doshomachi 2-chome Higashi-ku**
**Osaka-shi Osaka, 541 (JP)**

(72) Inventor: **Junichi, Azuma**
**26-201, 6 Kayando-cho**
**Nishinomiya Hyogo 662 (JP)**
Inventor: **Koichi, Watanabe**
**7-34, Aza-Shijuso, Kimokawa Inagawa-cho**
**Kawabe-gun Hyogo 666-02 (JP)**
Inventor: **Masatake, Hori**
**1 Andojicho 4-chome**
**Itami Hyogo 664 (JP)**

(74) Representative: **Geering, Keith Edwin et al**
**REDDIE & GROSE 16 Theobalds Road**
**London WC1X 8PL (GB)**

Courier Press, Leamington Spa, England.

## Description

The present invention relates to an apparatus for treatment of blood and more particularly to an apparatus for removing undesirable substances from blood plasma, and in which only the plasma is separated from a blood stream by a plasma separating membrane which prohibits the penetration of blood cellular components but allows the plasma to pass therethrough. In the apparatus the plasma is, after the removal of the undesirable substances, returned to the blood stream again through said plasma separating membrane.

Three methods of treating blood by the use of a blood treating material have been reported:

1. A direct hemoperfusion method (hereinafter called the "DHP method");

2. A plasma perfusion method (hereinafter called the "PP method"); and

3. A method of practicing the PP method by using a membrane-type plasma separator filled with a blood treating material (hereinafter called the "CPP method"). An example is described in U.S. Patent 4,013,564, and in Jinko Zoki, Vol. 9, No. 2, pp 506—509.

Of these methods, the DHP method has already been put in practice commercially. However this method, in which the blood is brought into direct contact with the blood treating material, has disadvantages, such as that it causes a decrease in blood platelets and white blood cells in the perfused blood, thrombogenesis, hemolysis, and microparticles of the blood treating material are sometimes released into the blood being treated. One countermeasure to these disadvantages is to coat the blood treating material with a substance which is highly biocompatible with the blood. However, in the present state of the technology, the use of such a coating is inevitably accompanied by a lowering of efficiency and an increase in the complexity of the process of manufacturing the blood treating material.

The PP method in which the blood itself is not brought into direct contact with the blood treating material, is free from such degeneration of the blood cellular components described above, but the apparatus, including the blood circuit, is complicated because this method requires a separate pump in the plasma circuit and a filter through which the treated plasma is returned to the blood stream. In addition, it is a further disadvantage of this method that a larger volume of extracorporeal circulating blood is required.

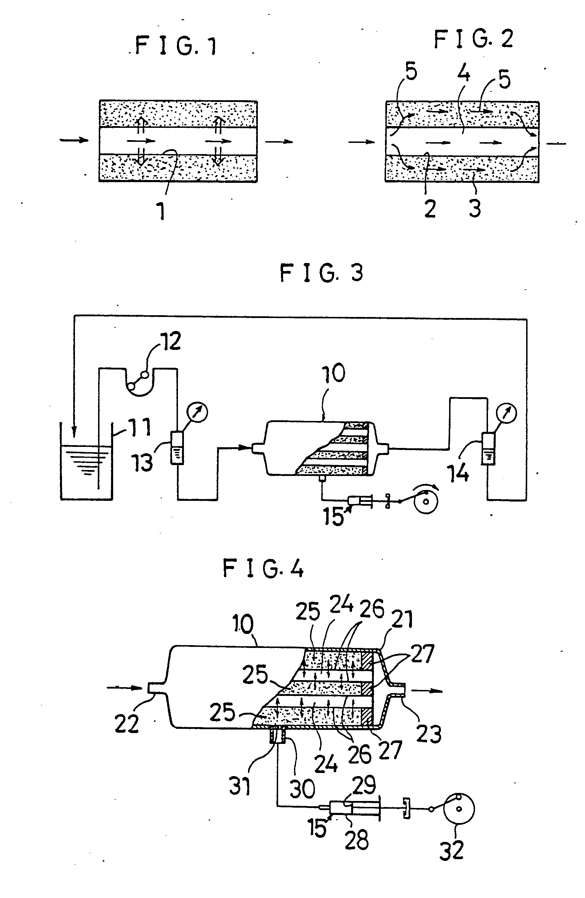

The CPP method not only is free from the problem of the direct contact between the blood and the blood treating material but also has an advantage that the problem of microparticles of the blood treating material being released into the blood is eliminated because the plasma separating membrane functions as a microfilter. Therefore, consideration has been given to putting this method into practice in two ways using either a hollow fiber membrane or a flat membrane. One way is to remove the undesired solutes by dispersing them through a so-called dialysing membrane 1, as shown schematically in Figure 1. The other way is to treat the plasma with a blood treating material, as is shown in Figure 2, causing only the plasma component 5 to separate from a blood stream path 4 through a high-flux membrane 2 which separates blood treating material 3 from the blood stream flow through the blood treating material 3, and then return to the blood stream. However, the former way, which is really outside the scope of the PP method, is not suitable for treating substances having a molecular weight higher than a medium value, while the latter way can hardly be expected to work effectively, if carried out in the way described, because the plasma permeation rate through the membrane is not constant and stable, but depends on the pressure resistance in the blood stream path 4 and the permeability of the membrane 2. Furthermore, the entire stream of the plasma has such a simple flow pattern that the plasma flows toward the blood treating material 3 in the vicinity of the blood stream entrance and returns to the original blood stream path 4 in the vicinity of the blood stream exit 4, as is indicated by arrows 5 in Figure 2, so that only about half the area of the membrane 2 carries out filtration in the vicinity of the entrance and the remaining half is used for the return of the treated plasma to the blood stream. Accordingly, in the region of the entrance of the blood stream path 4 there is formed a protein gel layer, which causes a substantial decrease of the effective area of the membrane 2, producing a serious disadvantage that a stable flow of blood is disturbed.

The present invention provides an apparatus for blood treatment, comprising:

a housing having a blood inlet means for introducing the blood to be treated and a blood outlet means for exhausting the treated blood from said housing;

at least one blood channel within said housing and extending from said inlet means to said outlet means and being constituted by a plasma-separating membrane capable of allowing the plasma component of blood to permeate therethrough while preventing the blood cells from penetrating therethrough, said housing having therein a space surrounding said membrane;

a blood treating material packed in the space within said housing around said blood channel; and

a pump connected to said inlet means for pumping blood into said inlet means at a predetermined pressure and pressure-varying means operatively associated with said housing for imposing from outside the housing on the space within the housing pressure changes, characterised in that said pressure-varying means are means for alternately changing the direction of the pressure difference between the inside of said blood channel and the blood treating material-containing space, whereby the plasma component of the blood flowing in said blood channel is caused to permeate through said plasma-separating membrane toward said blood treating material to bring the plasma into contact with said blood treating material and the plasma is thereafter caused to be returned to the blood channel through said plasma-separating membrane.

The direction of the pressure difference across the plasma separating membrane is preferably alternately forcibly changed by a simple mechanical pressure-varying means (e.g. a piston pump or a clamp mechanism) so as to effectively contact the plasma with the blood treating material. The pressure-varying means is preferably either an external mechanical device which alternatively raises and reduces the pressure in the space where the blood treating is packed, or a clamp operating on the external blood flow circuit connected with the outlet port of the blood treating unit.

Alternately changing the direction of the pressure difference across the plasma separating membrane may prevent formation of a protein gel layer and/or clogging of the pores of the membrane thus allowing the membrane to carry out stable filtration for a long time.

An apparatus according to the present invention preferably has a pressure-varying means, a blood pump and a blood treating unit which comprises a plurality of blood stream channels made of plasma-separating porous membrane with a blood treating material packed around the blood stream channels, both the blood stream channels and the blood treating material being encased in a housing having a blood inlet port and a blood outlet port. The blood to be treated, which is fed by the blood pump to the blood treating unit through the blood inlet, flows through the blood stream channels in the unit. While the blood is flowing through the channels, the pressure-varying means alternately changes the direction of the pressure difference between the space where the blood treating material is packed and the blood stream channels so as to cause the plasma component in the blood to permeate the plasma-separating membranes which form the blood stream channels reciprocally between the blood stream channels and the blood treating material. In such a manner, the plasma is cleaned by being brought into contact with the blood treating material, by adsorption from or by chemical or biological reaction with the blood treating material, and then caused to flow forcibly back into the blood stream channels.

US—A—4 071 444 and US—A—3 570 672 both disclose blood treatment apparatus having some of the features of the present invention, not disclosed in US—A—4 013 564, but these known apparatuses are not designed for separating plasma from the blood flowing into the blood channels and for reconstituting it after cleaning thereof. Moreover, the purpose of the oscillating transmembrane pressure gradient is to provide therewith a reciprocating pumping effect for the blood flowing in the blood channel, so as to avoid the use of a current blood pump, whereas in the present invention the problem solved by this alternating pressure is to prevent clogging of the membrane to improve its effectiveness. EP—A—0 052 004 discloses a similar apparatus to that disclosed in US—A—4 071 444 but improved by a system for controlling and monitoring the flow of blood. However, this apparatus is not devised so as to prevent clogging of the membrane, either.

The present invention will be described in greater detail by example only in connection with the accompanying drawings in which:

Figures 1 and 2 are schematic views showing examples of blood treating units of the prior art.

Figure 3 is a diagrammatic view of an example of a blood treating apparatus according to the present invention, wherein the blood treating unit is shown partially in cross-section;

Figure 4 is a partially cross-sectioned view on a larger scale of the blood treating unit of Figure 3;

Figure 5 is a view similar to Figure 3 showing another example of the blood treating apparatus according to the present invention;

Figure 6 is a view similar to Figure 4 showing the blood treating unit and a clamp mechanism of Figure 5;

Figure 7 is a schematic view showing a modified form of the clamp mechanism shown in Figure 6;

Figure 8 is a view similar in Figure 4 showing a modification of the embodiment of the blood treating unit of Figure 4;

Figure 9 is a view similar to Figure 5 showing a modification of the embodiment of the blood treating unit of Figure 5;

Figure 10 is a view similar to Figure 5 showing another modification of the blood treating unit shown in Figure 5;

Figures 11 to 16 are diagrammatic views of modified forms of apparatus in which the blood treating unit of the present invention can be used; and

Figures 17 to 20 are graphs showing experimental results obtained by the use of the apparatus of the present invention.

Figure 3 shows a blood treating apparatus in which the blood treating unit according to the present invention is used. The apparatus has a blood tank 11, a blood pump 12 connected to tank 11, a bubble trap 13, the blood treating unit 10, including pressure-varying means 15 and a further bubble trap 14 connected in series. The blood to be treated is fed by the blood pump 12 from the blood tank 11 to the blood treating unit 10 through the bubble trap 13. The blood fed to the blood treating unit 10 is cleaned there while passing therethrough, and is then returned to the blood tank 11 through the bubble trap 14. The blood tank may well be a human body.

The details of the first embodiment of the blood treating unit 10 used in the apparatus of Figure 3 are shown in Figure 4, together with an embodiment of the pressure-varying means 15 forming a part thereof. This embodiment of the blood treating unit comprises a housing 21 having a blood inlet port means 22, a blood outlet port means 23 and a pressure-introducing port 30. A known blood treating material 25 is contained within the housing 21 and a plurality of blood channels 24 is embedded in the blood treating material 25 extending from the inlet port means to the outlet port means. Each of the blood channels 24 is made of a tubular plasma-separating porous membrane 26 of a known material suitable for this purpose. The respective ends of each blood stream channel 24 are open to the blood inlet port means 22 and the blood outlet port means 23, and the blood treating material 25 adjacent to the port means is contained within the housing 21 by means of a wall 27 at the outlet side and a corresponding wall, not visible in the drawing, at the inlet side, so the material 25 is prevented from being mixed into the blood stream. The pressure-introducing port 30 opens into the blood treating material containing space around the channel 24 and is connected with the pressure-varying means which in this embodiment is a mechanical means in the form of a pump having a cylinder 28 and a piston 29 reciprocally movable therein by a piston driving mechanism 32, and a pressure-transmitting medium in the cylinder 28 is alternately pumped toward and drawn away from the blood treating material 25. A filter 31 is provided in the port 30. The pressure-transmitting medium can be physiological saline. The piston-driving mechanism 32 can be any known mechanism to drive a piston reciprocally.

In the thus constituted blood treating unit, the blood being transferred from a patient (corresponding to the blood tank 11 in Figure 3) is introduced into the blood treating unit via the inlet port means 22. As the blood introduced is passing through the blood channels 24, the piston 29 carries out more than one cycle of reciprocal movement to alternate the direction of the pressure difference between the blood channels 24 and the space containing the blood treating material 25 so as to cause the plasma component of the blood to permeate the plasma-separating membranes 25 first in one direction and then in the opposite direction as shown by the arrows. A withdrawal motion of the piston 29, i.e. to the right in Figure 4, effects a pressure decrease in the space where the blood treating material is packed to a pressure below the partial pressure of the plasma in the flowing blood, causing the plasma component of the blood in the blood channels 24 to be drawn into the blood treating material 25 through the whole area of the plasma-separating membranes 26. While the plasma thus drawn in is in contact with the blood treating material 25, the substances to be removed from the plasma are removed by adsorption from or by chemical or biological reaction with, the blood treating material 25. When the piston 29 is moved in the pressurizing direction, to the left in Figure 4, the pressure in the space where the blood treating material 25 is packed is made higher than that of the plasma in the blood channels 24. The plasma, which is now cleaned, is consequently pushed back through the membranes toward the blood channels 24 through the whole area of the plasma-separating membrane 26 to join the blood stream again. While the blood is passing through the blood channels 24, the above-described plasma movement through the plasma-separating membranes 26 is repeated one to several times as the occasion demands. In the present embodiment of the blood treating unit, the blood channels 24 can be constituted as capillaries made of hollow fibers. In this case the hollow fibers themselves act as the plasma-separating membranes 26, with the hollow interiors thereof serving as the blood channels 24. A bundle of a larger number of hollow fibers with a blood treating material packed between the fibers in the bundle can be used as a blood treating module having a large number of blood stream channels and a large surface area of the plasma-separating membrane. The plasma-separating membrane 26 is a porous membrane with a pore size, in general, of from 0,2 to 2,0 µm to prevent the blood cellular components from passing therethrough. However, a membrane with a smaller pore size can be used depending on the particle size of the blood treating material 25. The blood treating material 25 can be selected from among various adsorbents, enzymes and other conventional blood treating materials, depending on the kind of substances to be removed from the blood being treated. The blood treating unit can be provided with a plurality of pressure-introducing ports 30 to make the plasma movement smoother.

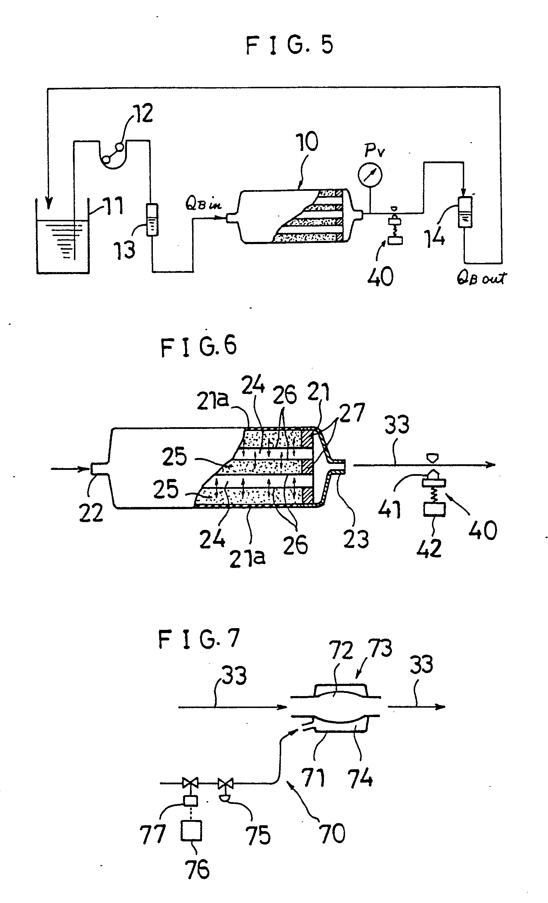

Figure 5 shows another blood treating apparatus, in which a clamp mechanism 40 is employed as the pressure-varying means of the blood treating unit. This apparatus, therefore, differs from that shown in Figure 3 in being provided with a clamp mechanism 40 instead of the pressure-varying pump 15 in Figure 3.

The details of the blood treating unit and the clamp mechanism 40 in Figure 5 are described with reference to Figure 6. In Figure 6, the housing 21 of the blood treating unit, the blood inlet port means 22 and the blood outlet port means 23, the blood treating material 25 and the plurality of blood channels 24 made of plasma-separating porous membranes, and the walls 27 are the same as described in connection with Figure 4. The only differences are that there is no pressure introducing port, such as the port 30, and at least a part or the whole of the housing wall which is in direct contact with the blood treating material 25 is made of an elastic material 21a such as silicon rubber, butyl rubber or soft poly-vinylchloride.

In the blood circuit 33 connected to the outlet port means 23 downstream of housing 21, there is provided a clamp mechanism 40 consisting of a throttle 41 and a timer 42 to actuate the throttle 41 intermittently. The throttle 41, which is for instance an electromagnetic valve, alternately restricts flow by partially closing or permits free flow by opening the circuit 33 at a predetermined time interval, being on-off controlled by the timer 42. The degree of throttling, which is freely adjustable, is preferably such as to cause in the circuit 33 a pressure $P_v$ which causes the plasma to permeate the plasma-separating membranes at the maximum filtration rate $Q_{Fmax}$ specific to the membranes. The circuit is not throttled completely, but it may be made to be completely throttled if a bypass is provided.

In this embodiment, the blood fed from a patient enters the housing 21 through the blood inlet port means 22, passes through the blood channels 24 constituted by the plasma-separating membranes 26 and returns to the patient through the circuit 33. In such a cyclic process of the blood flow, if the circuit 33 is throttled by the clamp mechanism 40, the pressure in the blood channels 24 is increased so that the plasma component flowing in the blood channels 24 is caused to permeate through the membranes 26 into the blood treating material 25 over the whole area of the plasma-separating membranes 26. In this case the pressure increase in the space in which the blood treating material 25 is packed is relieved by the expansion of the elastic material housing wall 21a so as to enable the plasma to flow continuously to the blood treating material-containing space. The plasma having filtered into the blood treating material-containing space comes into contact with the blood treating material 25 and is cleaned by adsorption or chemical or biological reaction. When the throttling of the circuit 33 discontinued, the restoring force of the elastic material 21a raises the pressure in the blood treating material-containing space sufficiently to cause the cleaned plasma to permeate back into the blood channels 24 again through the whole area of the plasma-separating membranes 26, so that the cleaned plasma joins the blood stream in the blood channels 24. While the blood is passing through the blood channels 24, the above-described plasma movement between the blood channels 24 and the blood treating material-containing space is repeated one to several times as the occasion demands. For instance, the number of repetitions is determined according to the restoring force of the elastic wall 21a of the housing. The elastic wall and the clamp mechanism together constitute the pressure varying means.

Figure 7 shows another embodiment 70 of the clamp mechanism. The clamp mechanism is constituted by a clamp unit 73 consisting of a soft inner tube 72 and a rigid outer case 71, which clamp unit is connected in the blood circuit 33. The rigid case 71 is provided with an air inlet port through which compressed air having the pressure controlled by a further valve 75 can be introduced into the space left around that inner tube 72 in the rigid outer case 71 to pinch the soft inner tube 72. A timer 76 and an electromagnetic valve 77 control the time and frequency of introduction of the compressed air and hence the pinching of the tube.

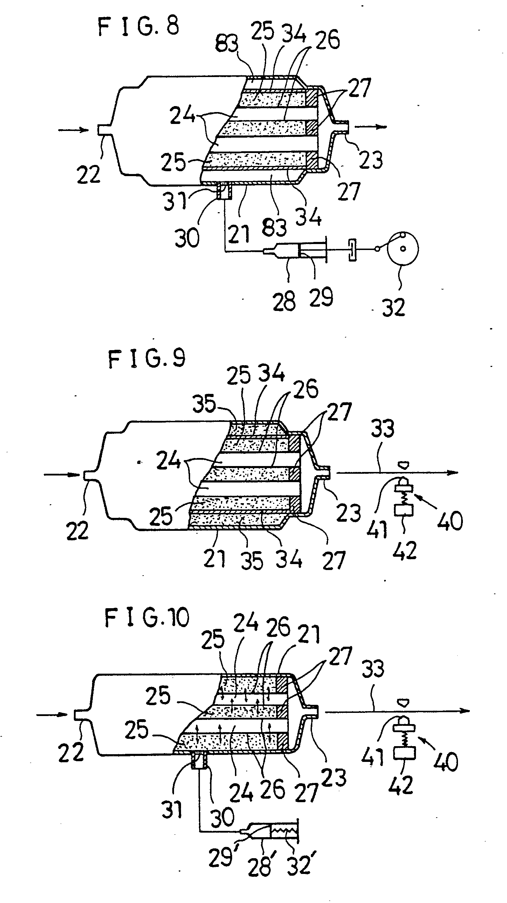

Figure 8 shows a further embodiment of the blood treating unit. In this embodiment, a pressure space 83 is provided around the blood treating material 25 enclosed by an elastic enclosure 34 within the housing 21 and which is impermeable to plasma and to pressure transmitting medium. The pressure space 83 is filled with a pressure-transmitting medium supplied through the pressure-introducing port 30 which is connected to the cylinder 28 of the external pressure-varying pump like that shown in Figure 4. A withdrawal motion of the piston 29 of the pressure-varying pump draws the pressure-transmitting medium out of space 83 effecting a pressure decrease in the pressure space 83. This pressure decrease is accompanied by a pressure decrease within the elastic enclosure 34 due to the expansion of the same. The pressure decrease within the elastic enclosure 34 causes the plasma component flowing in the blood channels 24 to filter through the plasma-separating membranes 26 into the space containing blood treating material 25. The blood treating material removes the waste contained in the plasma by adsorption or chemical or biological reaction. When the piston 29 moved in the pressurizing direction, the pressure-transmitting medium is forced into the pressure space 83, raising the pressure therein. The pressure increase in the pressure space 83 is transmitted through the elastic enclosure 34 to the space in which the blood treating material 25 is packed. The cleaned plasma is, therefore, caused to flow back into the blood channels 24 again through the whole area of the plasma-separating membranes 25. This embodiment has the advantage that any kind of liquid material can be used as the pressure-transmitting medium, because the blood treating material 25 is isolated from the pressure-transmitting material by means of the elastic enclosure 34.

The idea of introducing a pressure-transmitting medium into the housing can also be applied to an apparatus having a clamp mechanism such as is shown in Figure 5. Such an embodiment is shown in Figure 9. In this embodiment, an elastic material 35 is packed in the spaced formed between the housing 21 and the elastic enclosure 34 within which the blood treating material 25 is contained. The reversible filtering of the plasma through the plasma-separating membranes 25 is effected by the throttling action of the clamp mechanism 40 and by the substantially reversible elasticity of both the elastic enclosure 34 and the elastic packing 35.

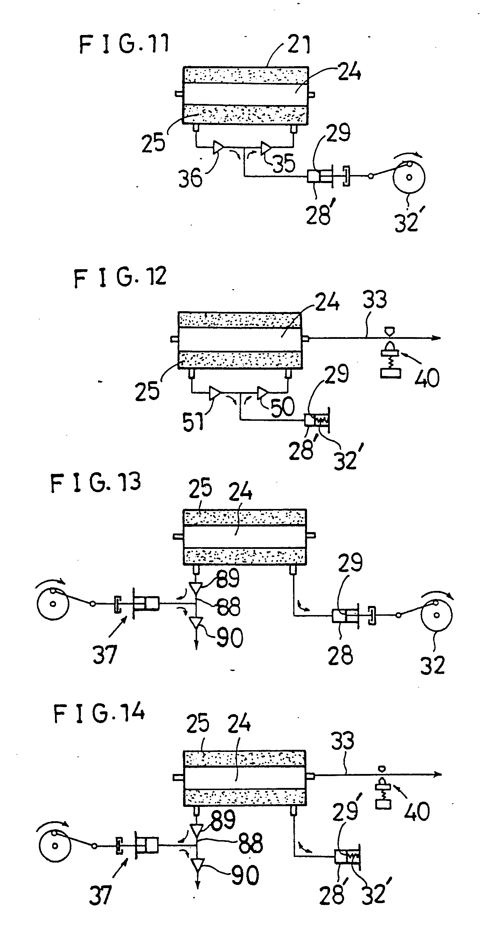

The embodiment shown in Figure 10 is a modification of the embodiment shown in Figure 6. This embodiment employs no elastic means in the housing 21, but employs an external piston mechanism consisting of a cylinder 28' and a piston 29' engaged by a spring 32' in the cylinder 28'. The cylinder 28' is connected with the housing 21 through the pressure-introducing port 30, enabling the plasma to flow through the filter 31 between the cylinder 28' and the space within which the blood treating material is packed. When the plasma filters through the plasma-separating membranes 26 into the blood treating material containing space at the time of the clamping action of the clamp mechanism 40, the plasma pushes the piston 29' to the right against the force of the spring 32' to accommodate the increased amount of plasma in the cleaning material containing space until the pressure of the plasma is balanced by the force of the spring. When the pressure in the blood channels 24 in reduced upon release of the clamping mechanism, the restoring force of the spring 32' which has been acting as means to counterbalance the pressure of the plasma, forces the plasma back to the blood channels 24. In this embodiment the spring 32' serves as the elastic means instead of the elastic packing or enclosure used in the embodiment shown in Figures 8 and 9. The spring 32' can be replaced with a compressible gas, such as air.

Figures 11 to 16, in which the housing 21, blood channels 24 and blood cleaning material 25 are shown schematically, show embodiments in which some attachments are incorporated.

The embodiment shown in Figure 11 employs separate pressure ports and two check valves 35 and 36 connected to make one the inlet pressure port and the other an outlet pressure port for the pressure-transmitting medium to flow in and out of the housing 21. The embodiment shown in Figure 12 is a modification of the embodiment shown in Figure 11, in which the pressure-varying pump is replaced with a clamp mechanism 40 and a pressure medium receiving spring loaded piston-cylinder mechanism 28', 29', 32' like that of Figure 10.

The embodiment shown in Figure 13 is provided with a fluid removing means. The water component of the plasma is removed by means of an exhaust pipe 88 connected with the blood treatment material containing space in which the blood treating material 25 is packed, two check valves 89 and 90 and a piston pump 37 connected between the check valves. The embodiment shown in Figure 14 is a modification of the embodiment shown in Figure 13, in which the pressure-varying pump is replaced with a clamp mechanism 40 and a pressure medium receiving spring loaded piston-cylinder mechanism 28', 29', 32'.

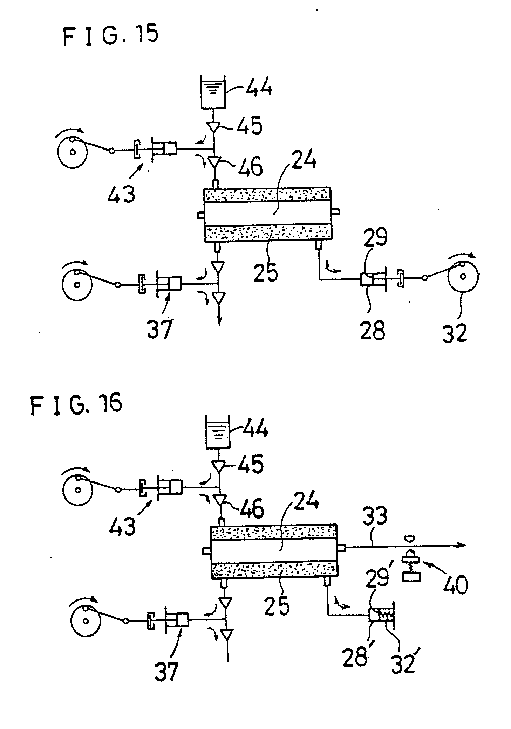

The embodiment shown in Figure 15 is provided with a fluid substituting means, in addition to said fluid removing means. The fluid in a tank 44 is supplied to the blood treating material 25 by means of two check valves 45, 46 and a piston pump 43 connected between the check valves. Figure 16 shows a modification of the embodiment shown in Figure 15 in which the pressure-varying pump is replaced by a clamp mechanism 40 and a pressure medium receiving spring loaded piston-cylinder mechanism 28', 29', 32'.

In some of the embodiments described above, a pressure-varying pump consisting of a piston and a cylinder is used as the pressure-varying means. However, the pressure-varying pump can of course be replaced with some other means, for instance, a roller pump which effects an increase and decrease in pressure according to the direction of its rotation.

Experimental results

The results of experiments with the apparatus according to the present invention will now be described.

A first experiment was conducted using the blood treating apparatus shown in Figures 3 and 4. The housing 21 of the blood treating unit 10 and an outer case of an SD-series artificial kidney manufactured by Takeda Chemical Industries with piping attached. The blood treating unit was constituted by a module consisting of said case, 2500 polypropylene hollow fibers (inner diameter: 330 μm max., pore diameter: 0,6 μm: manufactured by ENKA) as blood channels, and 50 gr of granular charcoal as a blood treating material. The effective total surface area of the blood channels, namely, the total surface area of the plasma-separating membranes was 0,5 m².

The blood used in the experiment was fresh bovine blood with a hematocrit value of 35%. In the blood, $VB_{12}$ and creatinine were supplied so that their concentrations were 20 mg/dl of plasma.

The experiment was carried out by circulating 2 liters of this blood in the blood apparatus shown in Figure 3 for 2 hours at a blood flow rate QB=100 ml/min for causing the charcoal to perform adsorption and using a pressure-varying pump. For reference a similar experiment was also performed without using the pressure-varying pump.

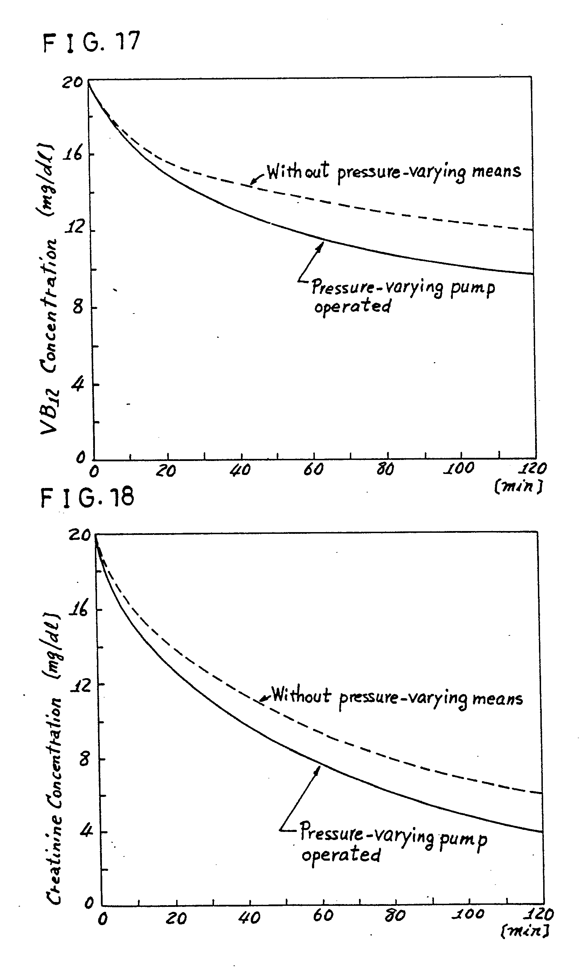

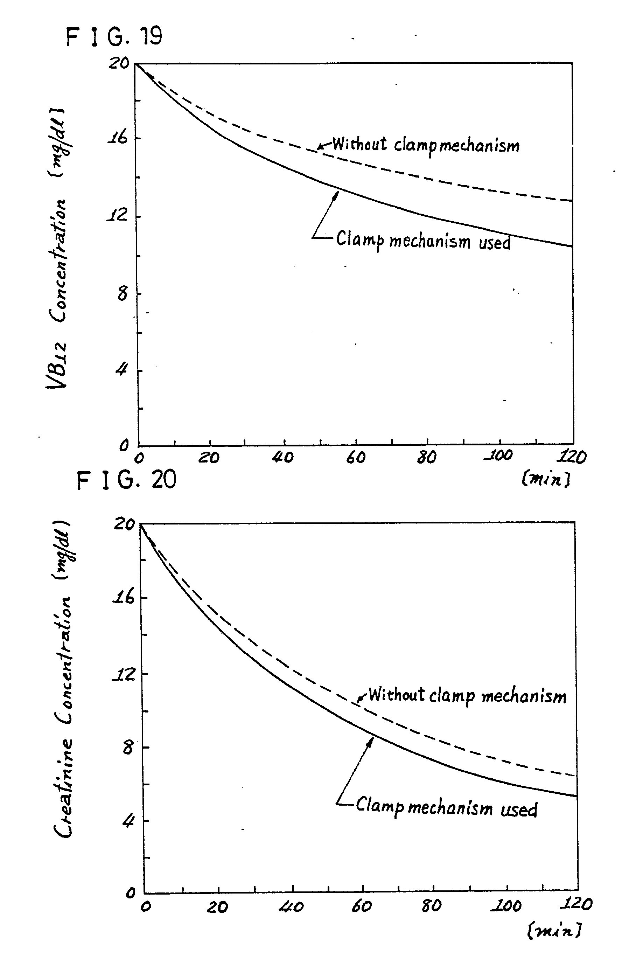

Figures 17 and 18 show the results for $VB_{12}$ and for creatinine, respectively. The solid lines in the Figures show the results of the first experiment, and the dotted lines those of the reference experiment in which a pressure-varying pump was not used. Figure 17 shows that the $VB_{12}$ concentration decayed to 60% of its original value in 55 minutes (solid line), which is shorter than a half of the 120 minutes needed for the same percentage decay in the reference experiment carried out without pressure-varying means. For the creatinine, Figure 18 shows that the apparatus according to the invention only takes 80 minutes for a 30% decay, while the apparatus without a pressure-varying means takes 120 minutes for the same decay. These facts show that the time needed for therapy can be greatly shortened.

Figures 19 and 20 show the results of an experiment performed with the apparatus shown in Figure 5 modified according to Figure 10 and using charcoal as a blood treating material. It was confirmed in advance that when fresh bovine blood with a hematocrit value of 35% flows at QB=100 ml/min, the maximum filtering rate $Q_{Fmax}$ of the membranes is about 35 ml/min and the pressure $P_{vmax}$ at the outlet 23 is about 35 mmHg (about 4,7 KPa). The valve 41, which is a kind of pinch valve, was adjusted so as to repeat throttling of a predetermined degree, being actuated by the electromagnetic means controlled by the timer 42. In this experiment the degree of throttling was adjusted to give an outlet pressure $P_v$=35 mmHg (about 4,7 KPa) at QB=65 ml/min. Under these conditions, the pressure at the outlet rises to 35 mmHg (about 4,7 KPa) when the valve 41 was throttled, and then the plasma filters into the blood treating material. After the duration of the throttling set on the timer 42 has expired, the pressure is relieved so that the piston 29' acted on by the restoring force of the spring 32' forces the plasma through the membranes into the blood channels 24 from the blood treating material containing space.

Under these conditions 2 liters of the fresh bovine blood (with a hematocrit value of 35%) containing both $VB_{12}$ and creatinine at the same concentration of 20 ml/dl was circulated for 2 hours at QB=100 ml/min and adsorption was performed by the charcoal. A reference experiment was also performed both for $VB_{12}$ and for creatinine but using no pressure varying means.

Figures 19 and 20 show the result for $VB_{12}$ and for creatinine, respectively, the solid lines showing the results of the apparatus according to the invention and the dotted lines the results of the reference experiment performed without a pressure-varying means. The times for reaching the levels of $VB_{12}$ and creatinine are substantially the same as for Figures 17 and 18.

The apparatus of the present invention, which can be made in a compact form, has a wide range of applications, and can be furnished with an apparatus for removing or for substituting biological fluids. In addition the present invention can be clinically applied as a useful therapeutical means for a patient suffering from acute hepatic insufficiency, immunological diseases or drug intoxication by using charcoal, immobilized enzymes, immunoadsorbents or other various adsorbents as the blood treating material.

## Claims

1. An apparatus for blood treatment, comprising:

a housing [21] having a blood inlet means [22] for introducing the blood to be treated and a blood outlet means [23] for exhausting the treated blood from said housing;

at least one blood channel [24] within said housing and extending from said inlet means [22] to said outlet means [23] and being constituted by a plasma-separating membrane [26] capable of allowing the plasma component of blood to permeate therethrough while preventing the blood cells from penetrating therethrough, said housing [21] having therein a space surrounding said membrane [26];

a blood treating material [25] packed in the space within said housing [21] around said blood channel [24]; and

a pump [12] connected to said inlet means [22] for pumping blood into said inlet means [22] at a predetermined pressure, and pressure-varying means [15] operatively associated with said housing [21] for imposing from outside the housing on the space within the housing pressure changes characterised in that said pressure-varying means [15] are means for alternately changing the direction of the pressure difference between the inside of said blood channel [24] and the blood treating material-containing space, whereby the plasma component of the blood flowing in said blood channel [24] is caused to permeate through said plasma-separating membrane [26] toward said blood treating material [25] to bring the plasma into contact with said blood treating material [25] and the plasma is thereafter caused to be returned to the blood channel through said plasma-separating membrane [26].

2. An apparatus as claimed in claim 1 wherein said pressure-varying means [15] is a mechanical pressure-varying means for alternately increasing and decreasing the pressure on said blood treating material.

3. An apparatus as claimed in claim 2 wherein said mechanical pressure-varying means [15] is a variable pressure pump connected to said housing [21] for alternately introducing into and withdrawing from said housing [21] a pressure-transmitting medium in order to cause the alternate increase and decrease of pressure on said blood treating material [25].

4. An apparatus as claimed in claim 3 wherein said variable pressure pump is connected to said housing [21] to open into said space containing the blood treating material.

5. An apparatus as claimed in claim 3 wherein said blood treating material [25] and said blood channel [24] are enclosed within an enclosure disposed within said housing, said enclosure being at least partly of an elastic material, the housing [21] also containing a space around said enclosure, said variable pressure pump being connected to said housing to open into said space around said enclosure.

6. An apparatus as claimed in claim 1 wherein said housing [21] has a blood circuit [33] connected to said outlet means [23], and said pressure-varying means is a clamp mechanism [40] acting on a soft tube of the blood circuit for intermittently throttling the flow of blood through said blood circuit [33] downstream of said outlet means [23].

7. An apparatus as claimed in claim 6 wherein at least part of said housing [21] is made of an elastic material [21a] and is in contact with said blood-treating material [25] for undergoing elastic expansion and contraction when said clamp mechanism [40] is operated.

8. An apparatus as claimed in claim 6 wherein said blood treating material [25] and said blood channel [24] are enclosed within an enclosure disposed within said housing, said enclosure being at least partly of an elastic material, the housing [21] also containing a space around said enclosure, said space being filled with an elastic packing material [35].

9. An apparatus as claimed in claim 6 wherein an elastic expansion and contraction means is provided outside said housing [21], said means consisting of a cylinder [28'] connected to said housing [21] to opening into said blood treating material-containing space, a piston [29'] reciprocally slidable in said cylinder [28'], and compressible means acting on said piston [29'] to counterbalance the pressure of the plasma in said blood-treating material space.

10. An apparatus as claimed in claim 9 in which said compressible means is a spring [32'].

11. An apparatus as claimed in claim 9 in which said compressible means is a compressible gas.

12. An apparatus as claimed in claim 6 wherein said clamp mechanism includes a timer [42] for controlling the intermittent throttling of the flow of blood.

**Patentansprüche**

1. Vorrichtung zur Blutbehandlung, bestehend aus

einem Gehäuse (21) mit einer Bluteinlaßeinrichtung (22) zum Zuführen des zu behandelnden Blutes und einer Blutauslaßeinrichtung (23) zum Abführen des behandelten Blutes aus dem Gehäuse;

mindestens einem Blutkanal (24) innerhalb des Gehäuses, der sich von der Einlaßeinrichtung (22) zu der Auslaßeinrichtung (23) erstreckt und aus einer Plasma-Abtrennmembran (26) besteht, die imstande ist, den Durchtritt der Plasmakomponente des Blutes zu gestatten, während sie die Blutkörperchen am Durchtritt hindert, wobei in dem Gehäuse (21) ein die Membran (26) umgebender Raum vorhanden ist;

einem Blutbehandlungsmaterial (25), das in den Raum gepackt ist, der den Blutkanal (24) innerhalb des Gehäuses (21) umgibt; und

einer Pumpe (12), die mit der Einlaßeinrichtung (22) verbunden ist, um Blut unter einem vorbestimmten Druck in die Einlaßeinrichtung (22) hineinzupumpen; sowie

einer Einrichtung (15) zum Variieren des Druckes, die mit dem Gehäuse (21) operativ verbunden ist, um vom Äußeren des Gehäuses der Druckänderungen auf den Raum innerhalb des Gehäuses aufzubringen,

dadurch gekennzeichnet, daß es sich bei der Einrichtung (15) zum Variieren des Druckes um eine Einrichtung zum abwechselnden Verändern der Richtung der Druckdifferenz zwischen dem Inneren des Blutkanals (24) und dem das Blutbehandlungsmaterial enthaltenden Raum handelt, wodurch die Plasmakomponente des in dem Blutkanal (24) strömenden Blutes veranlaßt wird, die Plasma-Abtrennmembran (26) in Richtung auf das Blutbehandlungsmaterial (25) zu durchdringen, damit das Plasma mit dem Blutbehandlungsmaterial (25) in Kontakt gebracht wird, und wodurch das Plasma anschließend veranlaßt wird, durch die Plasma-Abtrennmembran (26) hindurch in den Blutkanal zurückzukehren.

2. Vorrichtung nach Anspruch 1, dadurch gekennzeichnet, daß es sich bei der Einrichtung (15) zum Variieren des Druckes um eine mechanische Druckänderungseinrichtung handelt, die den auf das Blutbehandlungsmaterial einwirkenden Druck abwechselnd erhöht und vermindert.

3. Vorrichtung nach Anspruch 2, dadurch gekennzeichnet, daß es sich bei der mechanischen Druckänderungseinrichtung (15) um eine variable Druckpumpe handelt, die mit dem Gehäuse (21) in Verbindung steht, um ein Druckübertragungsmedium abwechselnd in das Gehäuse (21) einzuführen und aus ihm abzuziehen, um den abwechselnden Druckanstieg und -abfall in dem Blutbehandlungsmaterial (25) zu bewirken.

4. Vorrichtung nach Anspruch 3, dadurch gekennzeichnet, daß die variable Druckpumpe mit dem Gehäuse (21) verbunden und an den Raum angeschlossen ist, der das Blutbehandlungsmaterial enthält.

5. Vorrichtung nach Anspruch 3, dadurch gekennzeichnet, daß das Blutbehandlungsmaterial (25) und der Blutkanal (24) in einer Umschließung innerhalb des Gehäuses angeordnet sind, wobei die Umschließung mindestens zum Teil aus einem elastischen Material besteht und das Gehäuse (21) außerdem einen diese Umschließung umgebenden Raum enthält und wobei die variable Druckpumpe mit dem Gehäuse verbunden und an den die Umschließung umgebenden Raum angeschlossen ist.

6. Vorrichtung nach Anspruch 1, dadurch gekennzeichnet, daß an die Auslaßeinrichtung (23) des Gehäuses (21) ein Blutkreislauf (33) angeschlossen ist und daß es sich bei der Druckänderungseinrichtung um einen Klammermechanismus (40) handelt, der auf einen biegsamen Schlauch in dem Blutkreislauf wirkt, um den den Kreislauf (33) durchströmenden Blutstrom stromabwärts von der Auslaßeinrichtung (23) intermittierend zu drosseln.

7. Vorrichtung nach Anspruch 6, dadurch gekennzeichnet, daß mindestens ein Teil des Gehäuses (21) aus einem elastischen Material (21a) besteht und mit dem Blutbehandlungsmaterial (25) in Berührung steht, um beim Betätigen des Klammermechanismus (40) einer elastischen Ausdehnung und Zusammenziehung unterworfen zu werden.

8. Vorrichtung nach Anspruch 6, dadurch gekennzeichnet, daß das Blutbehandlungsmaterial (25) und der Blutkanal (24) in einer Umschließung innerhalb des Gehäuses angeordnet sind, wobei die Umschließung mindestens teilweise aus einem elastischen Material besteht und das Gehäuse (21) außerdem einen die Umschließung umgebenden Raum enthält, der mit einem elastischen Packungsmaterial gefüllt ist.

9. Vorrichtung nach Anspruch 6, dadurch gekennzeichnet, daß eine Einrichtung zum elastischen Ausdehnen und Zusammenziehen außerhalb des Gehäuses (21) angeordnet ist und aus einem Zylinder (28') besteht, der mit dem Gehäuse (21) in Verbindung steht und an den das Blutbehandlungsmaterial enthaltenden Raum angeschlossen ist, einem Kolben (29'), der in dem Zylinder (28') vor und zurück verschiebbar ist, sowie einer auf den Kolben (29') wirkenden zusammendrückbaren Einrichtung, um den Druck des Plasmas in dem das Blutbehandlungsmaterial enthaltenden Raum auszugleichen.

10. Vorrichtung nach Anspruch 9, dadurch gekennzeichnet, daß es sich bei der zusammendrückbaren Einrichtung um eine Feder (32') handelt.

11. Vorrichtung nach Anspruch 9, dadurch gekennzeichnet, daß es sich bei der zusammendrückbaren Einrichtung um ein zusammendrückbares Gas handelt.

12. Vorrichtung nach Anspruch 6, dadurch gekennzeichnet, daß zu der Klammereinrichtung ein Zeitschalter (42) gehört, der das intermittierende Drosseln des Blutstroms regelt.

## Revendications

1. Appareil pour le traitement du sang, qui comprend:

un réservoir (21) comportant une entrée du sang (22) servant à introduire le sang à traiter et une sortie du sang (23) pour évacuer le sang traité hors dudit réservoir;

au moins une canalisation pour le sang (24) à l'intérieur dudit réservoir, et, s'étendant depuis l'entrée (22) jusqu'à la sortie (23), une membrane (26) de séparation du plasma, capable de se laisser traverser par le composant plasma du sang, tout en empêchant les cellules sanguines d'y pénétrer, ledit réservoir (21) comportant à son intérieur un espace entourant ladite membrane (26);

une matière (25) de traitement du sang, garnissant l'espace à l'intérieur dudit réservoir (21) autour de ladite canalisation (24) pour le sang et

une pompe (12), raccordée auxdits organes d'entrée (22) pour pomper du sang vers ladite entrée (22) sous une pression prédéterminée et des moyens (15) pour faire varier la pression, associés fonctionnellement audit réservoir (29) pour imposer, à partir de l'extérieur du réservoir, des variations de pression dans l'espace à l'intérieur du réservoir,

caractérisé en ce que lesdits moyens (15) pour faire varier la pression sont des moyens pour modifier de façon alternative le sens de la différence de pression entre l'intérieur de ladite canalisation (24) pour le sang et l'espace contenant la matière de traitement du sang, ce qui fait que le composant plasma du sang s'écoulant dans ladite canalisation (24) à cet effet est amené à filtrer au travers de ladite membrane (26) de séparation du plasma en direction de ladite matière de traitement (25) pour venir en contact avec ladite matière (25), puis le plasma est amené à être renvoyé dans la canalisation pour le sang au travers de ladite membrane (26).

2. Appareil selon la revendication 1, dans lequel ledit moyen (15) pour faire varier la pression est un moyen mécanique de variation de la pression qui fait alternativement croître et décroître la pression sur ladite matière de traitement du sang.

3. Appareil selon la revendication 2, dans lequel ledit moyen (15) de modification mécanique de la pression est une pompe à pression variable raccordée audit réservoir (17) pour, alternativement, introduire dans ledit réservoir (21) et en extraire un fluide de transmission de la pression, afin de produire des augmentations et diminutions alternées de la pression sur ladite matière de traitement du sang (25).

4. Appareil selon la revendication 3, dans lequel ladite pompe à pression variable est reliée audit réservoir (21) pour déboucher dans ledit espace contenant la matière de traitement du sang.

5. Appareil selon la revendication 3, dans lequel ladite matière (25) de traitement du sang et ladite canalisation (24) pour le sang sont enfermés à l'intérieur d'une enveloppe disposée dans ladit boîtier, ladite enveloppe étant au moins en partie en matière élastique, le réservoir (21) contenant également un espace libre autour de ladite enveloppe, ladite pompe à pression variable étant reliée audit réservoir pour déboucher dans ledit espace situé autour de ladite enveloppe.

6. Appareil selon la revendication 1, dans lequel ledit réservoir (21) comporte un circuit de sang (33) connecté à la sortie (23) et ledit moyen pour faire varier la pression est un mécanisme de pincement (40) agissant sur un tube souple du circuit de sang pour étrangler de façon intermittente l'écoulement du sang passant au travers dudit circuit (33) pour le sang, en aval de ladite sortie (23).

7. Appareil selon la revendication 6, dans lequel une partie au moins dudit réservoir (21) est faite en matière élastique (21a) et est en contact avec ladite matière (25) du traitement du sang pour subir des dilatations et contractions élastiques lorsque ledit mécanisme de pincement (40) est actionné.

8. Appareil selon la revendication 6, dans lequel ladite matière (25) de traitement du sang et ladite canalisation (24) pour le sang sont enfermés à l'intérieur d'une enveloppe disposée dans ledit réservoir, ladite enveloppe étant, au moins en partie, en matière élastique, le réservoir (21) contenant également un espace situé autour de ladite enveloppe, ledit espace étant rempli d'une matière de bourrage élastique (35).

9. Appareil selon la revendication 6, dans lequel un moyen élastique de dilatation et de contraction est prévu à l'extérieur dudit réservoir (21), ledit moyen étant constitué par un cylindre (28') relié audit réservoir (21) pour déboucher dans ledit espace contenant la matière de traitement du sang, un piston (29') pouvant coulisser en va-et-vient dans ledit cylindre (28') et des moyens compressibles agissant sur ledit piston (29') pour contrebalancer la pression du plasma, dans ledit espace, de la matière de traitement du sang.

10. Appareil selon la revendication 9, dans lequel ledit moyen compressible est un ressort (32').

11. Appareil selon la revendication 9, dans lequel ledit moyen compressible est un gaz compressible.

12. Appareil selon la revendication 6, dans lequel ledit mécanisme de pincement inclut un minuteur (42) pour commander l'étranglement intermittent de l'écoulement du sang.

# FIG.1

# FIG.2

# FIG.3

# FIG.4

F I G. 5

F I G. 6

F I G. 7

FIG. 8

FIG. 9

FIG. 10

FIG.11

FIG.12

FIG.13

FIG.14

FIG. 15

FIG. 16

F I G. 17

F I G. 18

F I G. 19

F I G. 20